# EUROPEAN PATENT APPLICATION

(11) **EP 2 002 790 A2**
(43) Date of publication of application: **17.12.2008**
(21) Application number: 06832419.3
(22) Date of filing: 30.10.2006
(51) Int. Cl.: A61B 16/00

(54) **TOXIC GAS EXPOSURE PREVENTING APPARATUS FOR DISSECTION PRACTICE ROOM**

(30) Priority: 22.03.2006 JP 2006078035
(71) Applicant: KOKEN LTD., Chiyoda-ku Tokyo 102-0081 (JP)
(72) Inventor: KUBOTA, Yuji, Tokyo 102-0081 (JP); FUJISHIRO, Yuki, Tokyo 102-0081 (JP)
(74) Representative: Clark, Jane Anne
(86) International application number: PCT/JP2006/322062
(87) International publication number: WO 2007/108160

(57) **Abstract**

A toxic gas exposure preventing system for an anatomic practice room is provided which not only can obtain a satisfactory toxic gas exposure preventing effect without impairing workability but also permits the use of existing dissecting tables as they are without requiring any special work such as an exhaust duct installing work.

A push hood having a uniform air flow blow-off mechanism and a pull hood having an air flow suction/exhaust mechanism are disposed independently of each of the dissecting tables in such a manner that at least a portion of a uniform air flow passes in contact with a donor body on the dissecting table and is thereafter sucked into the pull hood. Further, a filter having a toxic gas adsorbing function is installed within the pull hood.

## Description

### Field of the Invention

The present invention relates to a toxic gas exposure preventing ventilation system for an anatomic practice room. Particularly, the present invention is concerned with a system for preventing the exposure of toxic gas (e.g., formaldehyde gas) issuing mainly from an antiseptic such as formalin when conducting an anatomic practice for a donor body, i.e. specimen, having been subjected to an antiseptic treatment using formalin for example.

### Background of the Invention

An anatomic practice performed by medical students or the like is called a medicine faculty type anatomic practice. In this practice, plural donor bodies having been subjected to an antispectic treatment using formalin for example are usually dissected simultaneously on plural dissecting tables in a practice room. Since this practice is performed in the presence of many persons and for a long time, it is necessary that toxic gas, e.g., formaldehyde gas, evolved from donor bodies be removed efficiently and economically.

Generally, such toxic gas has so far been removed by operating a ventilation fan, opening a window, or using an air cleaner. With these means, however, a satisfactory exposure preventing effect is not obtained. Among newly-built anatomic practice rooms there are included those equipped with central management type air conditioning equipment having an intake port and an exhaust port. However, equipment capable of removing toxic gases is expensive in both equipment cost and maintenance cost and thus a wide spread of such equipment is difficult. In view of this point, various local exhaust ventilation means for each dissecting table have been proposed (see JP 2004-000451A, JP 2003-320220A, JP 2003-116859A, and JP 2001-061909A). However, such local ventilation means involve various problems such as, for example, the structure thereof obstructing a practicing work, the application to existing dissecting tables being difficult and the toxic gas exposure preventing effect for students taking practice being unsatisfactory.

### Disclosure of the Invention

### Objects of the Invention

It is an object of the present invention to provide a toxic gas exposure preventing system for an anatomic practice room which system can attain a satisfactory toxic gas exposure preventing effect without impairing the workability of students taking practice and which permits the use of existing dissecting tables as they are without requiring any special work such as installing an exhaust duct.

### Summery of the Invention

The present invention firstly resides in a toxic gas exposure preventing system for an anatomic practice room, characterized in that a push hood having a uniform air flow blow-off mechanism, i.e. supply uniform air flow mechanism, and a pull hood having an air flow suction mechanism are disposed for each of plural dissecting tables in the anatomic practice room and independently of each of the dissecting tables in such a manner that at least a portion of a uniform air flow passes in contact with a donor body on the dissecting table and is thereafter sucked into the pull hood.

The present invention secondly resides in the above system wherein a filter having a toxic gas adsorbing function is installed within the pull hood.

The present invention thirdly resides in the above system wherein the filter installed within the pull hood is positioned lower than the air flow suction mechanism and a cleaned air flow is exhausted into the room from a position lower than the dissecting table.

The present invention fourthly resides in the above system wherein a filter having a toxic gas adsorbing function is installed also within the push hood.

The present invention fifthly resides in the above system wherein the height of an air flow blow-off opening surface of the push hood and/or the height of an air flow suction opening surface of the pull hood are (is) adjustable.

The present invention sixthly resides in the above system wherein the height adjustment is made by a double structure of the opening surface(s) or using a shielding plate(s) disposed vertically movably in the opening surface(s).

The present invention seventhly resides in the above system wherein the height adjustment is made by a height adjusting base portion(s) underlying the pull hood and/or the push hood.

The present invention eighthly resides in the above system wherein the push hood and the pull hood are disposed opposedly to each other at both ends in a longitudinal direction of each of the dissecting tables.

The present invention ninthly resides in the above system wherein one push hood is disposed at a position higher than each of the dissecting tables and a pair of pull hoods are disposed opposedly to each other at least outside both ends in a longitudinal direction of each of the dissecting tables, thereby allowing a uniform air flow to pass as a descending flow.

The present invention tenthly resides in the above ninth system wherein a pair of pull hoods are disposed opposedly to each other also at both ends in a transverse direction of each of the dissecting table.

### Effects of the Invention

By using the toxic gas exposure preventing system of the present invention it is possible to effectively prevent the exposure of toxic gas such as formaldehyde gas by using an extremely small air flow without impairing the workability of students taking practice. Besides, the device of the present invention can be installed and moved easily while using existing anatomic practice room and dissecting tables as they are without the need of such a work as installing an exhaust duct. Further, it is possible to ensure a high air conditioning efficiency.

### Brief Description of the Drawings

Fig. 1 is an explanatory diagram showing an example of installation of a toxic gas exposure preventing system according to the present invention.
Fig. 2 is an explanatory diagram showing an example of a push hood having a height adjusting function.
Fig. 3 is an explanatory diagram showing an example of opening surfaces of a double structure.
Fig. 4 is an explanatory diagram showing an example of shielding plates disposed on an opening surface.
Fig. 5 is an explanatory diagram showing another example of a toxic gas exposure preventing system according to the present invention.
Fig. 6 is an explanatory diagram showing an example of a pull hood used in the installation example of Fig. 5.

### Preferred Embodiments of the Invention

The present invention will be described hereinunder with reference to the drawings.

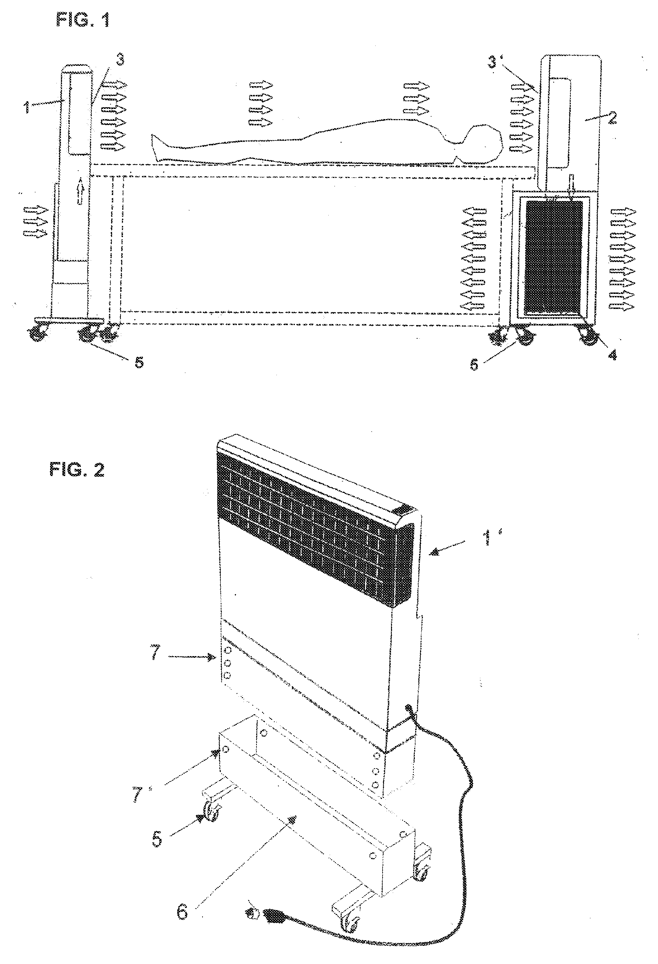

According to the present invention, a predetermined push-pull ventilation system is installed for each of dissecting tables in an anatomic practice room. As to in what manner the push-pull ventilation system is to be installed, one of the following two installation methods if roughly classified may be adopted in the present invention. It is Fig. 1 that illustrates the whole of the first embodiment of installation method and it is Fig. 2 that illustrates the whole of the second embodiment of installation method.

First, a description will be given about the first embodiment.

The first embodiment concerns a horizontal flow or obliquely downward flow type. As shown in Fig. 1, a push hood 1 as an air blow-off hood is disposed on a donor body foot-side of each dissecting table, while a pull hood 2 as an air suction hood is disposed on a donor body head side of the same table, or vice versa. It is preferable that the hood height be as low as possible so as not to obstruct a practicing work, provided the hood height is preferably higher than the height of the donor body on the table. The push hood may be somewhat raised upward so that an air flow is blown off obliquely downward.

Arrows indicate air flowing directions. A uniform air flow is blown off as a horizontal flow from the push hood 1 toward the pull hood 2. By the uniform air flow is meant a state in which the magnitude of the flow velocity is substantially constant anywhere in its section when the flow is seen in terms of a section perpendicular to the flow. Here, a state in which variations in velocity distribution in the absence of any obstacle is within ±30%, preferably ±20%, of a mean value indicates the uniform air flow.

If the push hood 1 has an air flow blow-off opening at a position higher than each dissecting table, there may be used a suitable conventional push hood. However, it is preferable to adopt such a configuration as illustrated in the figure wherein the whole is a vertical thin plate-like structure and the indoor air is taken in from a lower portion on the side opposite to the air blow-off opening.

As to the pull hood, like the conventional pull hood, it has an exhaust fan and makes the indoor ventilation possible. It is preferable that a filter for adsorbing toxic gas such as formaldehyde gas issued from a donor body be installed within the pull hood used in the present invention. The filter may be a suitable conventional filter insofar as it can remove toxic gas typical of which is formaldehyde gas. Also as to the push hood, there sometimes is a case where it is preferable that a filter for adsorbing toxic gas such as formaldehyde be installed within the push hood, although the necessity thereof is smaller than that for the pull hood. For preventing flue dust or the like from getting inside and entangled with a fan in the system or for preventing clogging of a perforated plate such as punching metal which is for creating a uniform flow, it is preferable that a course dust filter be disposed in an air intake port of the push hood. Likewise, it is preferable to dispose a course dust filter in the pull food.

The filter-installed position within the pull hood 2 may be inside an opening surface 3' located in an upper portion of the pull hood. However, as shown in the figure, it is preferable that the interior of the pull hood 2 be formed as an upper, lower, two-stage structure and that a fan be disposed in the upper stage and a filter receptacle portion 4 be provided in the lower stage. As the filter there may be used a known filter capable of removing toxic gas typified by formaldehyde gas. But, particularly, an arranged structure of plural sheets, say, 5 to 10 sheets, of activated charcoal filters is preferred. As shown in the figure, the lower portion of the pull hood having the filter receptacle portion is larger in required size than the upper portion having an air flow sucking function, so it is preferable that a part of the pull hood be positioned below the dissecting table to save the space.

It is preferable that exhaust be done through the entire surface of the lower portion of the pull hood (arrows indicating air flowing directions, i.e., exhaust directions into the room, are described as only right and left directions in the figure and forward and backward arrows are omitted).

In the conventional toxic gas exposure preventing systems for an anatomic practice room, exhaust is generally outdoor exhaust, but in the system of the present invention, since very clean air resulting from filtration of toxic gas through the filter can be exhausted in a relatively small amount, it becomes possible to effect indoor exhaust and hence possible to attain the reduction in size of the system and space-saving without affecting the air conditioning performed by an air conditioner or the like and without the need of laying pipes or the like.

Moreover, indoor exhaust can be done in four directions from the lower portion of the pull hood, and even when it is necessary to install the pull hood in contact with a wall surface, it is possible to effect exhaust in the remaining three directions. Consequently, in comparison with a like system permitting exhaust in only one direction, the amount of exhaust air flowing near the feet of apprentices can be decreased to a completely inappreciable extent for the apprentices.

Preferably, the push hood and the pull hood are each provided with casters 5 as shown in the figure to permit easy movement thereof.

Preferably, the area of the air flow blow-off opening surface of the push hood and that of the pull hood are equal to each other, or the latter is the larger.

Preferably, a vertical adjusting mechanism for adjustment to an appropriate position (height) is provided for each of the push hood and the pull hood because all the dissecting tables are not always equal in height or a certain donor body may be very big. As the vertical adjusting mechanism there may be used any of various mechanisms.

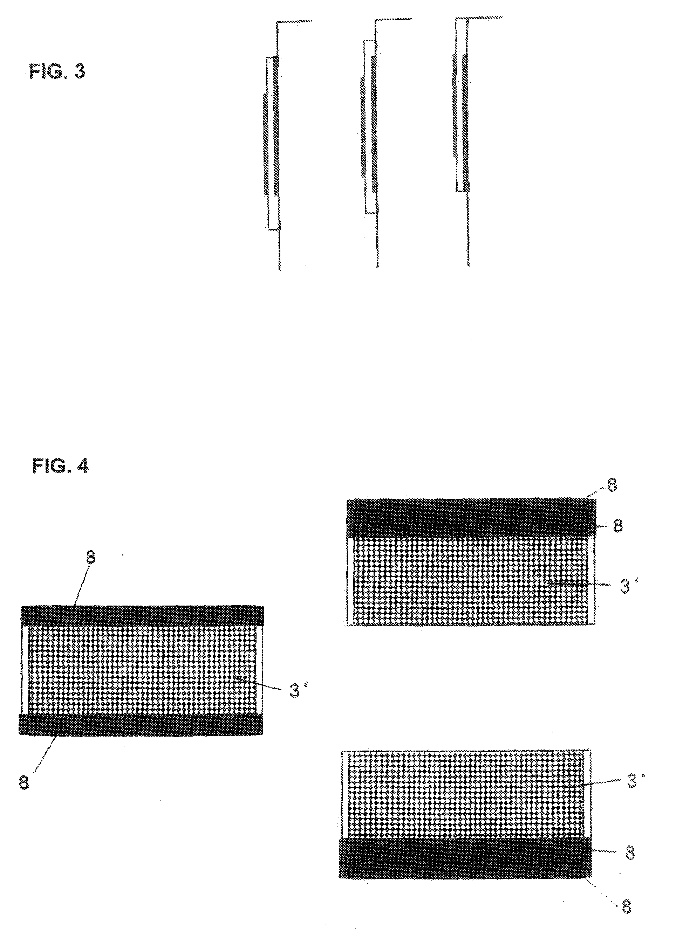

Examples of height adjusting mechanisms are shown in Figs. 2 to 4. Fig. 3 shows an example of opening surfaces formed as a double structure. In this double structure, if the opening surface on the front side is made larger than the opening surface on the inner side, the range corresponding to the difference in size can be made an operation range. The same figure shows a state in which the opening surface on the front side is made vertically movable and the height thereof has been adjusted to lower, middle and upper stages successively from the left side. It is preferable for the opening surface to have a perforated body such as punching metal. According to this method, it is not necessary to move the body itself vertically with respect to each of the push hood and the pull hood and therefore the height adjustment can be done very easily. Besides, obstruction to the visual field can be kept to a minimum because the height of the body does not change.

On the other hand, there also is a case where the push hood is to be installed at an obliquely upward position for creating an obliquely downward flow. In this case, the legs of the body of the push hood may be provided with a vertical adjusting mechanism or a separate table provided with a vertical adjusting mechanism may be added to the body. Such a vertical adjusting mechanism may also be used for the pull hood. By so doing, it becomes possible to create a more appropriate air flow and carry out ventilation effectively.

Fig. 2 shows an example in which the body itself is endowed with a high adjusting function. More specifically, the push hood is composed of a push hood body 1' and a base portion 6 easily separable from each other. Further, plural height adjusting holes 7 are formed vertically in both ends of a lower portion of a push hood body 1' and height adjusting holes 7' are formed also in both ends of the base portion 6 which is box-shaped so as to permit fitting therein of the lower portion of the push hood body 1'. With fixing members such as bolts, the push hood body 1' is fixed at a predetermined height.

On the other hand, as to the pull hood, it is preferable that a vertically movable shielding plate be disposed on the air flow sucking opening surface. This mode is shown in Fig. 4. In the example shown in Fig. 4, two elongated shielding plates 8 are disposed at upper and lower ends, respectively. When the opening surface is to be positioned lower than the shielding plates, the two shielding plates are moved to the upper end side, while when the opening surface is to be positioned higher than the shielding plates, the two shielding plates are moved to the lower end side. The number of the shielding plates may be one or three or more. In the embodiment of using a shielding plate(s), it is preferable that the total area of the opening surface of the pull hood be made larger by about 10% to 30% than that of the push hood and that a part or the whole of the opening surface be closed with the shielding plate(s). As to the material of the shielding plate(s), there may be used a shielding plate(s) of a suitable material, e.g., metallic or plastic plate(s).

In the first embodiment described above, both push hood and pull hood are disposed on both head side and foot side outside a donor body on each dissecting table and are not disposed in the longitudinal direction of the dissecting table, thus causing no obstacle to students taking practice.

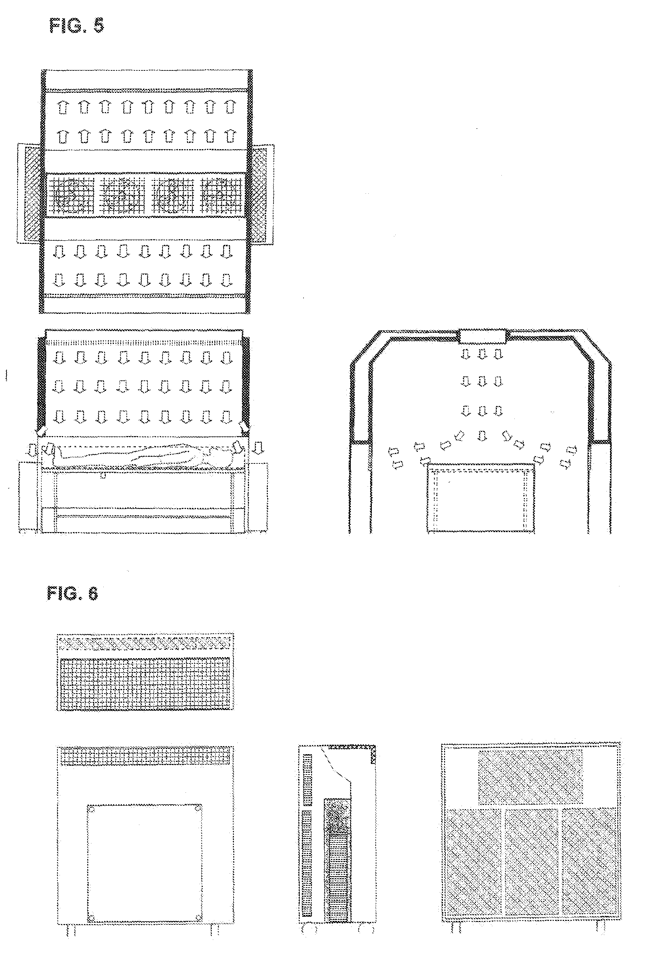

The second embodiment of the present invention concerns a descending flow type. In this second embodiment, a push hood having a uniform air flow blow-off mechanism is disposed above each dissecting table and a total of two or four pull hoods are disposed in two opposed longitudinal directions or in four longitudinal and transverse directions of the dissecting table. Fig. 5 shows a typical example thereof. As shown in the same figure, a push hood having a uniform air flow blow-off mechanism is disposed above the dissecting table and a pair of pull hoods are disposed in the transverse direction of the dissecting table in contact with or somewhat spacedly from the dissecting table in such a manner that openings are positioned at approximately the same height. In case of installing the pull hoods somewhat spacedly from the dissecting table, it is necessary that the amount of sucked air be made somewhat large.

On the other hand, in the longitudinal direction of the dissecting table, students taking practice perform operations in a stand-up state in many cases and there is a fear that the sheet which wraps the donor body may close the opening surfaces. For this reason, it is not preferable to dispose the push hoods so as to be in contact with the dissecting table. In this case, as shown on the right upper side in Fig. 5, a pair of pull hoods should be disposed so as to leave a space enough for passing of students taking practice. Preferably, each opening portion is provided with a height adjusting mechanism so that the height thereof can be adjusted in accordance with the height of the dissecting table. As to the pull hoods disposed in the longitudinal direction, there may be used a divided structure to match the length of the dissecting table.

A uniform air flow leaving the push hood is divided in four directions near the dissecting table, but preferably the divided flows are sucked in by four pull hoods and toxic gas such as formaldehyde gas is exhausted.

The pull hoods in the transverse direction may be omitted. In this case, it is preferable to lengthen the whole of each pull hood in the longitudinal direction to a sufficient extent or increase the air volume.

Within each pull hood there are installed a fan and a filter for removing toxic gas such as formaldehyde as in the first embodiment. In this case, a duct for exhaust to the exterior is not needed at all. Besides, because of interior exhaust, there is no air conditioning load. Also as to the push hoods, it is preferable that the same filter as in the first embodiment be disposed in each push hood.

Also in case of disposing pull hoods in the longitudinal direction of each dissecting table in the second embodiment of the present invention, since the pull hoods are disposed spacedly from the working position of the students taking practice, the pull hoods scarcely obstruct the work. Although toxic gas-containing air also moves toward the working position, there is little exposure of the toxic gas to the students taking practice in the presence of the push hoods and pull hoods properly positioned.

By positioning as above the pull foods in the longitudinal direction of each dissecting table and by setting the height of the pull hoods in the transverse direction of each dissecting table almost equal to the height of the dissecting table, it is possible to prevent the pull hoods from obstructing the work of the students taking practice. Moreover, by constructing the pull hoods movably and by separation thereof from the dissecting table, it is possible to further improve the workability.

With the ordinary type of pull hoods alone, it is impossible to fully suck in gas evolved at a position spaced away from the hoods and it is very likely that the students taking practice will be exposed to the gas. However, by providing a uniform air flow from above, it is possible to suppress the rise of toxic gas such as formaldehyde gas and hence possible to greatly diminish the possibility of exposure to toxic gas of the students taking practice.

When smoke was allowed to rise as a substitute for toxic gas with use of the system shown in Fig. 1 (the opening surface area of the push hood and the pull hood = 750 mm x 300 mm, distance between the two: 200 cm) and at a blow-off air velocity in the push hood of 0.4 m/sec, it turned out that all the smoke was in effect sucked by the pull hood.

## Claims

1. A toxic gas exposure preventing system for an anatomic practice room, **characterized in that** a push hood having a uniform air blow-off mechanism and a pull hood having an air flow suction mechanism are disposed for each of plural dissecting tables in the anatomic practice room and independently of each of the dissecting tables in such a manner that at least a portion of a uniform air flow passes in contact with a donor body on the dissecting table an is thereafter sucked into the pull hood.

2. The system of claim 1, wherein a filter having a toxic gas adsorbing function is installed within the pull hood.

3. The system of claim 2, wherein the filter installed within said pull hood is positioned lower than the air flow suction mechanism and a cleaned air flow is exhausted into the room from a position lower than the dissecting table.

4. The system of claim 2 or claim 3, wherein a filter having a toxic gas adsorbing function is installed also within the push hood.

5. The system of any of claims 1 to 4, wherein the height of an air flow blow-off opening surface of the push hood and/or the height of an air flow suction opening surface of the pull hood are (is) adjustable.

6. The system of claim 5, wherein the height adjustment is made by a double structure of the opening surface(s) or using a shielding plate(s) disposed vertically movably in the opening surface(s).

7. The system of claim 5 or claim 6, wherein the height adjustment is made by a height adjusting base portion(s) underlying the pull hood and/or the push hood.

8. The system of any of claims 1 to 7, wherein the push hood and the pull hood are disposed opposedly to each other at both ends in a longitudinal direction of each of the dissecting tables.

9. The system of any of claims 1 to 7, wherein one push hood is disposed at a position higher than each of the dissecting tables and a pair of pull hoods are disposed opposedly to each other at least outside both ends in a longitudinal direction of each of the dissecting tables, thereby allowing a uniform air flow to pass as a descending flow.

10. The system of claim 9, wherein a pair of pull hoods are disposed opposedly to each other also at both ends in a transverse direction of each of the dissecting tables.
